# FASCICULE DE BREVET EUROPEEN

(11) **EP 0 776 636 B1**
(45) Date de publication et mention de la délivrance du brevet: **06.09.2000**
(21) Numéro de dépôt: 96420342.6
(22) Date de dépôt: 28.11.1996
(51) Int. Cl.: A61B 17/86

(54) **Dispositif pour la fixation d'une prothèse et notamment d'une prothèse glénoidienne de l'omoplate**
Vorrichtung zur Prothesenfixierung, insbesondere einer Schulterblattgelenkpfannenprothese
Fixing device for a prosthesis, especially for a glenoid prosthesis of a shoulder blade

(30) Priorité: 30.11.1995 FR 9514454
(43) Date de publication de la demande: 04.06.1997
(73) Titulaire: TORNIER SA, 38330 Saint-Ismier (FR)
(72) Inventeur: Walch, Gilles, 69003 Lyon (FR); Boileau, Pascal, 06300 Nice (FR)
(74) Mandataire: Myon, Gérard Jean-Pierre

(56) Documents cités:
- EP-A- 0 196 409
- EP-A- 0 393 543
- EP-A- 0 495 340
- FR-A- 2 615 726
- FR-A- 2 653 659
- US-A- 3 805 775
- US-A- 4 091 806
- US-A- 4 167 047

## Description

La présente invention a trait à un dispositif permettant la fixation d'une prothèse dans l'os d'un patient et plus particulièrement la fixation d'une prothèse glénoïdienne de l'omoplate.

On connaît des dispositifs de fixation comportant soit des vis, soit un liant tel que du ciment pour le maintien d'un élément prothétique contre de l'os.

On connaît, dans le cas des prothèses glénoïdiennes, des dispositifs comportant une assise métallique qui est fixée sur l'os par des vis ou par des plots d'ancrage cannelés. Les vis de fixation permettent de mettre l'os en compression sous l'assise métallique de la prothèse.

Par contre, lorsque l'assise métallique comporte des plots d'ancrage, ces derniers ne permettent pas la mise en compression de l'os.

Les plots d'ancrage cannelés peuvent être creux et fendus et recevoir à l'intérieur, après avoir été introduits dans les trous osseux, une pièce femelle qui augmente légèrement le diamètre des plots afin d'exercer une contrainte sur l'os sans le déformer. Ainsi, l'ancrage de l'assise métallique de la prothèse est obtenu par gonflement de la géométrie initiale des plots d'ancrage.

Lorsque l'assise métallique comporte des vis de fixation, on constate que leur mise en place n'est pas aisée et qu'il existe un risque de migration des vis par tassement de l'os, entraînant l'éjection de l'insert en matière plastique constituant la surface articulaire.

Par contre, lorsque l'assise métallique est solidaire de plots d'ancrage, on constate que leur mise en place est plus facile, mais ils ont pour inconvénients de ne pas mettre l'os en compression sous la prothèse, entraînant une déstabilisation de cette dernière dans le temps.

On connaît, par la demande de brevet FR-A-2 615 726, un élément de fixation prothétique à plot d'ancrage dans lequel une vis peut être insérée dans un plot d'ancrage. L'écartement des ailettes n'est pas efficacement contrôlé car il a lieu uniquement grâce à leur élasticité. Les ailettes doivent donc avoir une faible rigidité. Or, lorsqu'une prothèse doit être mise en place dans un os dur, tel qu'une omoplate, l'utilisation d'ailettes de faible rigidité n'est pas adaptée.

C'est à ces inconvénients qu'entend plus particulièrement remédier la présente invention.

La présente invention a pour but de concevoir une assise métallique comportant des moyens déformables élastiquement pour comprimer de l'os spongieux entre la partie corticale et l'assise métallique, ces moyens pouvant être rigides sur toute leur longueur.

Le dispositif de fixation suivant la présente invention comprend une assise métallique pourvue d'un plateau solidaire d'au moins une cheville d'ancrage constituée d'ailettes déformables élastiquement et au moins une vis coopérant avec un écrou qui traverse la cheville, pour écarter lesdites ailettes dans une position éloignée de l'axe longitudinal de ladite cheville, ladite cheville d'ancrage comportant un alésage interne présentant, au niveau d'une embase cyclindrique de raccordement de ladite cheville audit plateau, un logement circulaire de plus grand diamètre que ledit alésage interne, ledit logement permettant d'écarter lesdites ailettes tout en gardant leur rigidité sur toute leur longueur.

Grâce à l'invention, l'écartement des ailettes est obtenue par la coopération de l'écrou et de la vis alors que les ailettes peuvent être réalisées dans un matériau rigide car elles se déforment uniquement au niveau du logement circulaire de plus grand diamètre, c'est-à-dire dans leur portion de plus faible épaisseur.

En outre, le dispositif de fixation comporte au moins une cheville pourvue d'au moins trois ailettes délimitées par des rainures qui sont parallèles à l'axe longitudinal de ladite cheville.

Le dessin annexé, donné à titre d'exemple, permettra de mieux comprendre l'invention, les caractéristiques qu'elle présente et les avantages qu'elle est susceptible de procurer :
- Fig. 1 est une vue en perspective éclatée illustrant le dispositif de fixation suivant la présente invention.
- Fig. 2 et 3 sont des vues partielles montrant le dispositif de fixation avant l'écartement des ailettes de la cheville d'ancrage.
- Fig. 4 et 5 sont des vues partielles identiques à celles des fig. 2 et 3, mais représentant la cheville d'ancrage dans une position écartée.
- Fig. 6 et 7 sont des coupes montrant l'encliquetage de l'insert en matière plastique sur l'assise métallique du dispositif de fixation suivant la présente invention.
- Fig. 8 et 9 sont des coupes semblables à celles de fig. 9 et 10, mais représentant une variante de fixation de l'insert sur l'assise métallique.

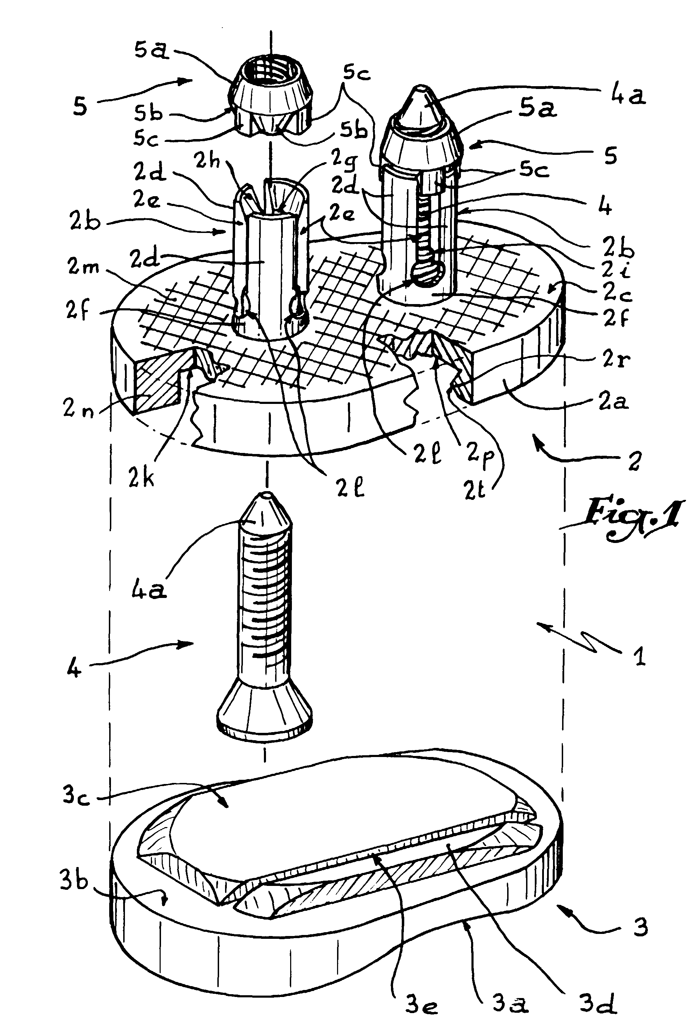

On a représenté en fig. 1 un dispositif de fixation 1 comportant une assise métallique 2 destinée à recevoir un insert 3 en polyéthylène et notamment un implant de prothèse glénoïdienne de l'omoplate.

L'assise métallique 2 est constituée d'un plateau 2a qui est solidaire d'au moins une cheville d'ancrage 2b s'étendant perpendiculairement à la face d'appui 2c dudit plateau.

Le cheville d'ancrage 2b comporte des ailettes 2d qui sont délimitées par des rainures 2e parallèles à l'axe longitudinal de ladite cheville. Cette dernière comprend trois ailettes 2d régulièrement réparties sur sa périphérie. Les ailettes 2d de la cheville 2b se raccordent à la face d'appui 2c du plateau 2 par une embase cylindrique 2f indéformable et commune à chaque ailette.

On note que les rainures 2e débouchent chacune dans un espace de plus grand diamètre 2l se trouvant à proximité de l'embase cylindrique indéformable 2f. La longueur des rainures 2e est suffisante pour empêcher la déformation de l'embase cylindrique 2f.

La cheville 2b comporte un alésage interne 2g qui présente une entrée conique 2h l'extrémité libre de chaque ailette 2d. A l'opposé de l'entrée conique 2h et plus particulièrement au niveau de l'embase cylindrique 2f, l'alésage interne 2g comporte un logement circulaire 2i de plus grand diamètre. L'alésage 2g de la cheville 2b débouche dans une empreinte conique 2j ménagée sur la face opposée 2k à celle 2b du plateau 2a. L'empreinte conique 2i et l'alésage 2g sont prévus pour recevoir une vis 4 qui coopère avec un écrou 5.

La vis 4 présente une extrémité libre à profil conique 4a améliorant la pénétration de la cheville 2b dans l'os.

L'écrou 5 comporte deux pans 5a, 5b qui sont inclinés dans des directions opposées. Le pan incliné 5b comprend sur son pourtour des ergots 5c en relief régulièrement espacés. Les ergots 5c sont espacés de manière à correspondre avec les rainures 2e de la cheville 2b lors du serrage de la vis 4 dans l'écrou 5.

On note que le pan 5a est d'une inclinaison identique au profil conique 4a de la vis 4 pour constituer, lors de la mise en place de l'assise métallique contre l'os, un profil continu et pointu améliorant la pénétration de la cheville 2b.

Le pan 5b présente en outre une inclinaison semblable à celle de l'entrée conique 2h de la cheville 2b prévue sur chaque ailette 2d.

La face d'appui 2c du plateau 2a présente un réseau d'empreintes ou de saillies 2m obtenu par usinage ou par fonderie. Les empreintes ou saillies 2m présentent la forme de trous non débouchants à profil en goutte d'eau.

On a représenté en fig. 2 la mise en place de l'assise métallique 2 contre la couche dure 6a d'un os 6. La couche dure ou corticale 6a est percée préalablement d'au moins un trou 6b permettant le passage de la cheville 2b correspondante qui est associée à une vis 4 et un écrou 5 pour que cet ensemble pénètre à l'intérieur de la couche spongieuse 6c. On note que la cheville 2b pénètre facilement à l'intérieur de la couche spongieuse 6c, étant donné que l'écrou 5 et plus particulièrement son pan incliné 5a, se trouve dans le prolongement du profil conique 4a de la vis 4.

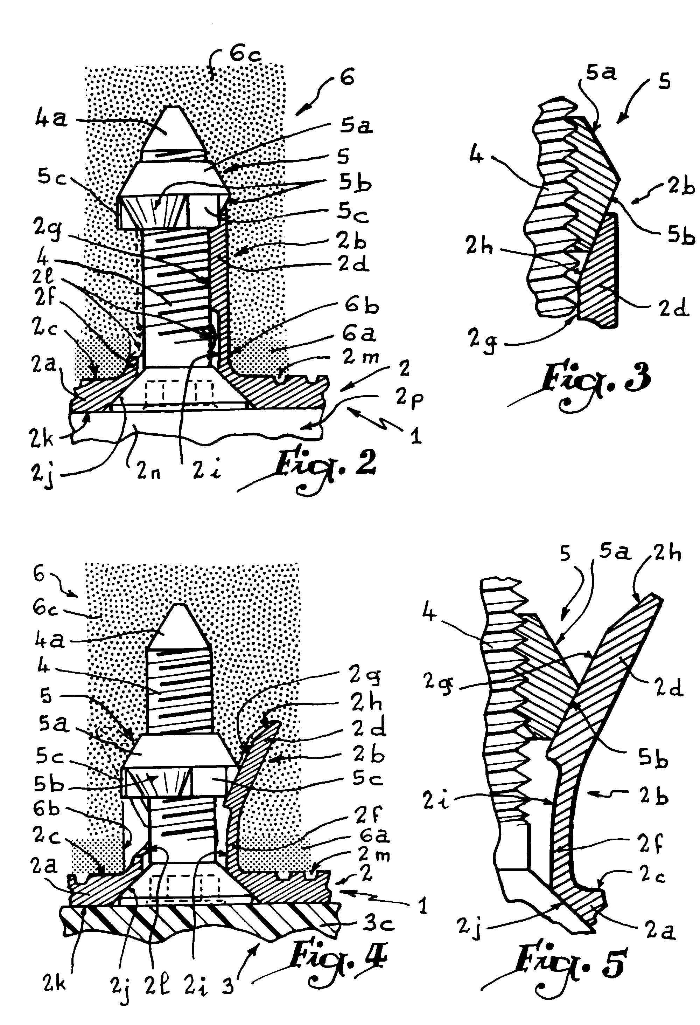

On remarque que l'écrou 5 et plus particulièrement son pan incliné 5b est en contact avec l'entrée conique 2h de chaque ailette 2d de la cheville 2b (fig. 3). Dans cette position, on garantit que les ergots 5c de l'écrou 5 coopèrent avec les rainures 2e de la cheville 2b pour éviter que cet écrou tourne dans le vide lorsque le chirurgien procèdera au serrage de la vis 4.

En fig. 4, on a représenté l'assise métallique 2 définitivement immobilisée contre l'os 6 d'un patient. Cette immobilisation définitive est obtenue par le serrage de la vis 4 dans son écrou 5, permettant à ce dernier du fait de son profil de pénétrer entre les ailettes 2d en vue de les écarter en direction éloignée de l'axe de la cheville 2b. On constate que le pan incliné 5b de l'écrou 5 vient en appui contre la face de l'alésage interne 2g de chaque ailette 2d après leur déformation pour empêcher toute translation verticale de la vis 4 à l'intérieur de l'empreinte conique 2i évitant l'éjection de l'insert 3 en matière plastique (fig. 4 et 5).

Ainsi, la vis 4 est bloquée en translation par l'écrou 5 d'un côté du plateau 2a et par sa tête de l'autre côté. Cette disposition permet de protéger l'insert en matière plastique 3 réalisant la surface articulaire contre toute migration supérieure de la vis 4, susceptible d'éjecter l'insert de l'assise métallique 2a.

Les ailettes 2d de la cheville 2b permettent dans leurs positions écartées de comprimer la couche spongieuse 6c de l'os 6 entre lesdites ailettes et la couche dure ou corticale 6a améliorant la solidité de la fixation de l'assise métallique 2.

Lors de la pénétration de l'écrou 5 à l'intérieur des ailettes 2d de la cheville 2b, on remarque que l'embase cylindrique 2f ne subit aucune déformation pour éviter la rupture de la couche dure ou corticale 6a lors du serrage. En effet, le logement 2i de plus faible épaisseur que celle prévue pour les ailettes 2d permet à ces dernières de se déformer au niveau du raccordement avec ledit logement en gardant leur rigidité sur toute leur longueur.

Dans la position de fig. 4, les ailettes 2d permettent d'appuyer la face 2b du plateau 2a contre la couche dure 6a de l'os 6. Le chirurgien préalablement au serrage de la vis 4 dans l'écrou 5, rapporte, dans les empreintes 2m de la face 2b, de l'os spongieux prélevé dans l'humérus, préalablement préparé dans ce type d'intervention.

La face 2b est revêtue par le fabricant d'une fine couche d'hydroxylapatite favorisant la repousse osseuse qui solidifie l'ancrage au cours du temps.

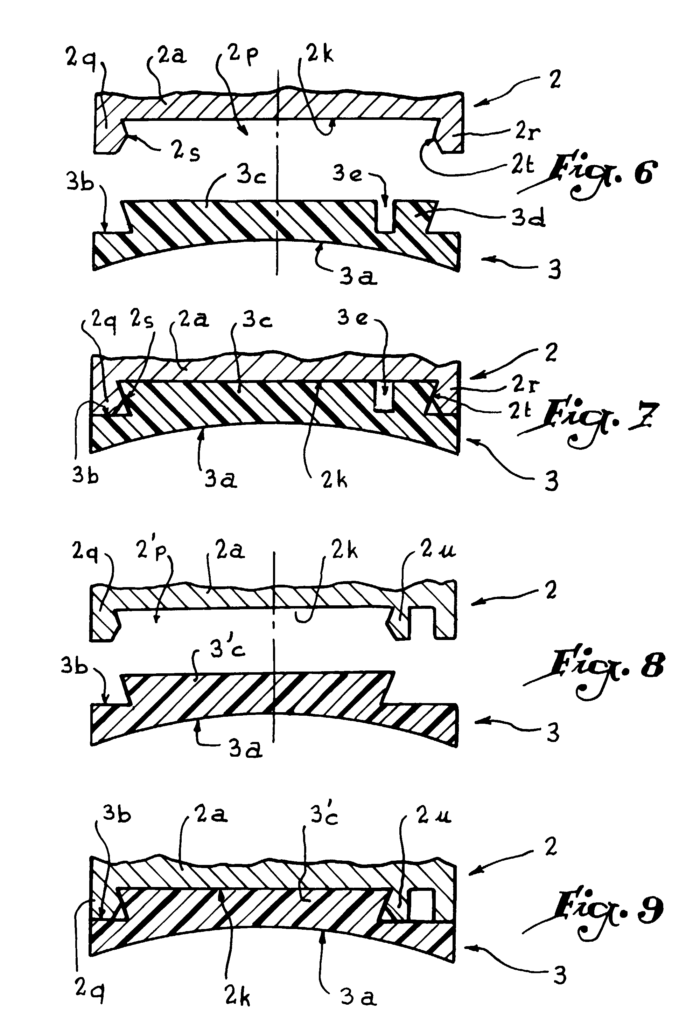

En fig. 6 et 7, on a représenté une première variante de fixation de l'insert 3 sur l'assise métallique 2 du dispositif de fixation 1.

La face supérieure 2k du plateau 2 est munie d'un rebord 2n qui délimite une cuvette 2p ouverte vers le haut. Le rebord 2n possède deux tronçons 2g, 2r parallèles présentant chacun des nervures 2s, 2t orientées vers l'intérieur de la cuvette 2p.

L'insert plastique ou céramique 3 comprend une surface articulaire 3a, tandis que son contour est identique à celui de l'assise métallique 2. L'insert 3 comporte sur sa face inférieure 3b opposée à celle articulaire 3a, une partie en saillie 3c de même contour que la cuvette 2p et de hauteur égale à la profondeur de ladite cuvette afin d'être engagée dans cette dernière. Une lèvre 3d est prévue parallèle à l'un des bords de la saillie 3c pour permettre l'encliquetage de l'insert 3 à l'intérieur de la cuvette 2p et entre les rebords 2n. La lèvre 3d est obtenue grâce à une rainure 3e ménagée dans l'assise 3c de l'insert 3 impliquant une certaine souplesse à ladite lèvre.

Il est possible de prévoir d'autres configurations pour la fixation de l'insert 3 sur l'assise métallique 2 du dispositif de fixation 1. En fig. 8 et 9, on a montré une seconde variante de fixation de l'insert 3 qui est symétriquement opposée de celle représentée en fig. 6 et 7. En effet, l'assise métallique 2 comporte à l'intérieur d'une cuvette 2'p une lèvre souple 2u permettant l'encliquetage de l'insert, tandis que ce dernier présente une partie en saillie 3'c de forme complémentaire à celle de la cuvette 2'p.

On note que le dispositif de fixation 1 peut comprendre un nombre de chevilles 2b qui varie suivant le cas opératoire. Chaque cheville 2b peut présenter un nombre d'ailettes variable suivant le diamètre de la cheville.

Comme il ressort de ce qui précède, l'invention apporte une grande amélioration à l'état de la technique en proposant un dispositif de fixation 1 facile d'utilisation assurant la cohésion des différents composants dans le temps, avec un ancrage très solide, et assurant de plus une mise en compression de l'os 6 sous le plateau 2a.

## Revendications

1. Dispositif pour la fixation d'une prothèse contre la couche dure d'un os, caractérisé en ce qu'il comprend une assise métallique (2) pourvue d'un plateau (2a) solidaire d'au moins une cheville d'ancrage (2b) constituée d'ailettes (2d) déformables élastiquement et au moins une vis (4) coopérant avec un écrou (5) qui traverse la cheville (2b), pour écarter lesdites ailettes (2d) dans une position éloignée de l'axe longitudinal de ladite cheville (2b), ladite cheville d'ancrage (2b) comportant un alésage interne (2g) présentant, au niveau d'une embase cyclindrique (2f) de raccordement de ladite cheville (2b) audit plateau (2a), un logement circulaire (2i) de plus grand diamètre que ledit alésage interne, ledit logement permettant d'écarter lesdites ailettes (2d) tout en gardant leur rigidité sur toute leur longueur.

2. Dispositif suivant la revendication 1, caractérisé en ce que ladite embase cylindrique (2f) est indéformable.

3. Dispositif suivant la revendication 1, caractérisé en ce que la cheville (2b) comporte au moins trois ailettes (2d) délimitées par des rainures (2e) qui sont disposées parallèlement à l'axe longitudinal de ladite cheville, tandis que les rainures (2e) sont prévues d'une longueur suffisante pour empêcher la déformation de l'embase cylindrique (2f).

4. Dispositif suivant la revendication 1, caractérisé en ce que la cheville (2b) présente une entrée conique (2h) disposée à chaque extrémité libre des ailettes (2d).

5. Dispositif suivant la revendication 1, caractérisé en ce que l'écrou (5) présente deux pans (5a, 5b) d'inclinaison différente, de manière que l'un (5b) des pans inclinés comporte sur son pourtour des ergots en relief (5c) régulièrement répartis.

6. Dispositif suivant la revendication 5, caractérisé en ce que les ergots (5c) sont disposés sur la périphérie du pan incliné (5b) de l'écrou (5) de manière coopérer avec les rainures (2e) de la cheville (2b) correspondante.

7. Dispositif suivant la revendication 5, caractérisé en ce que l'un (5a) desdits pans a une inclinaison identique à un profil conique (4a) d'extrémité de ladite vis.

8. Dispositif suivant la revendication 1, caractérisé en ce que le plateau (2a) comporte une face d'appui (2c) solidaire d'au moins une cheville (2b) et présentant un aspect de surface (2m) contitué d'un réseau d'empreintes ou de saillies.

9. Dispositif suivant la revendication 1, caractérisé en ce que le plateau (2a) est destiné à recevoir sur la face opposée (2k) à celle (2c) un insert (3) réalisé en une matière plastique ou céramique comprenant une surface articulaire (3a).

10. Dispositif suivant la revendication (9) caractérisé en ce que l'insert (3) est encliqueté dans une cuvette (2p, 2'p).

11. Dispositif suivant la revendication (10), caractérisé en ce que la cuvette (2p) est délimitée par un rebord (2n) possédant deux tronçons parallèles (2g, 2r) qui présentent chacun des nervures (2s, 2t) orientées vers l'intérieur de la cuvette (2p)

12. Dispositif suivant la revendication (10), caractérisé en ce que l'insert (3) comporte une partie en saillie (3c) et une lèvre souple (3d) pour son encliquetage à l'intérieur de la cuvette (2p) du plateau (2a).

13. Dispositif suivant la revendication (10), caractérisé en ce que l'insert (3) comporte une partie en saillie (3'c) de forme complémentaire à celle de la cuvette (2'p), tandis que ladite cuvette (2'p) présente une lèvre souple (2u) pour l'encliquetage de l'insert (3).

## Claims

1. A device for fixing a prosthesis against the hard layer of a bone, characterized in that it comprises a metal foundation (2) provided with a plate (2a), which is integral with at least one anchoring pin (2b) made of elastically deformable wings (2d), and at least one screw (4) which co-operates with a nut (5) and passes through the pin (2b), to spread said wings (2d) into a position away from the longitudinal axis of said pin (2b), said anchoring pin (2b) having an internal bore (2g) having, at the level of a cylindrical base (2f) connecting said pin (2b) to said plate (2a), a circular housing (2i) of larger diameter than said internal bore, said housing allowing said wings (2d) to spread, while retaining their rigidity over their entire length.

2. A device according to Claim 1, characterized in that said cylindrical base (2f) is non-deformable.

3. A device according to Claim 1, characterized in that the pin (2b) has at least three wings (2d) delimited by slots (2e) which are disposed parallel to the longitudinal axis of said pin, while the slots (2e) are of sufficient length to prevent deformation of the cylindrical base (2f).

4. A device according to Claim 1, characterized in that the pin (2b) has a conical entrance (2h) disposed at each free end of the wings (2d).

5. A device according to Claim 1, characterized in that the nut (5) has two faces (5a, 5b) of different inclination, so that one (5b) of the inclined faces has raised lugs (5c) distributed evenly over its circumference.

6. A device according to Claim 5, characterized in that the lugs (5c) are disposed over the periphery of the inclined face (5b) of the nut (5) in such a way as to co-operate with the slots (2e) of the corresponding pin (2b).

7. A device according to Claim 5, characterized in that one (5a) of said faces is inclined identically to a conical end profile (4a) of said screw.

8. A device according to Claim 1, characterized in that the plate (2a) has a bearing surface (2c) integral with at least one pin (2b) and having a surface aspect (2m) consisting of a network of recesses or projections.

9. A device according to Claim 1, characterized in that the plate (2a) is designed to receive on the face (2k) opposite the bearing surface (2c) an insert (3) made of a plastic or ceramic material comprising an articular surface (3a).

10. A device according to Claim 9, characterized in that the insert (3) engages in a tray (2p, 2'p).

11. A device according to Claim (10), characterized in that the tray (2p) is delimited by a rim (2n) having two parallel sections (2g, 2r), each of which has ribs (2s, 2t) oriented towards the interior of the tray (2p).

12. A device according to Claim (10), characterized in that the insert (3) has a projecting portion (3c) and a flexible lip (3d) for its engagement inside the tray (2p) of the plate (2a).

13. A device according to Claim (10), characterized in that the insert (3) has a projecting portion (3'c) having a shape which is complementary to that of the tray (2'p), while said tray (2'p) has a flexible lip (2u) for engagement of the insert (3).

## Patentansprüche

1. Vorrichtung zur Prothesenfixierung an einer harten Knochenschicht,
**gekennzeichnet** durch
ein metallisches Grundelement (2), das mit einer Platte (2a) versehen ist, die mit mindestens einem Verankerungsdübel (2b) fest verbunden ist, der aus elastisch verformbaren Flügeln (2d) besteht,
und durch mindestens eine Schraube (4), die mit einer Mutter (5) zusammenwirkt und den Verankerungsdübel (2b) durchsetzt, um die Flügel (2d) in eine Position auseinander zu spreizen, die von der Längsachse des Verankerungsdübels (2b) beabstandet ist, wobei der Verankerungsdübel (2b) eine Innenbohrung (2g) aufweist, die auf Höhe eines zylindrischen Sockels (2f) zur Verbindung des Verankerungsdübels (2b) mit der Platte (2a) eine kreisförmige Aufnahme (2i) mit einem größeren Durchmesser als demjenigen der Innenbohrung (2g) vorgesehen ist, und wobei die Aufnahme (2i) es gestattet, die Flügel (2d) unter Beibehaltung deren Steifigkeit über deren gesamte Länge auseinander zu spreizen.

2. Vorrichtung nach Anspruch 1,
dadurch **gekennzeichnet,**
daß der zylindrische Sockel (2f) undeformierbar ist.

3. Vorrichtung nach Anspruch 1,
dadurch **gekennzeichnet,**
daß der Verankerungsdübel (2b) mindestens drei Flügel (2d) aufweist, die durch Nuten (2e) begrenzt sind, die parallel zur Längsachse des Verankerungsdübels (2b) angeordnet sind, wobei die Nuten (2e) auf einer Länge vorgesehen sind, die ausreicht, um die Deformation des zylindrischen Sockels (2f) zu verhindern.

4. Vorrichtung nach Anspruch 1,
dadurch **gekennzeichnet,**
daß der Verankerungsdübel (2b) einen kegelförmigen Einschnitt (2h) aufweist, der an jedem freien Ende der Flügel (2d) angeordnet ist.

5. Vorrichtung nach Anspruch 1,
dadurch **gekennzeichnet,**
daß die Mutter (5) zwei Flächen (5a, 5b) unterschiedlicher Neigung aufweist derart, daß die eine geneigte Fläche (5b) auf ihren äußeren Umfang in regelmäßiger Verteilung abstehende Nocken (5c) aufweist.

6. Vorrichtung nach Anspruch 5,
dadurch **gekennzeichnet,**
daß die Nocken (5c) auf dem Umfang der geneigten Flächen (5b) der Mutter (5) derart angeordnet sind, daß sie mit den Nuten (2e) des entsprechenden Verankerungsdübels (2b) zusammenwirken.

7. Vorrichtung nach Anspruch 5,
dadurch **gekennzeichnet,**
daß die eine der geneigten Flächen (5a) eine Neigung aufweist, die identisch einem kegelförmigen Profil (4a) des Endes der Schraube (4) ist.

8. Vorrichtung nach Anspruch 1,
dadurch **gekennzeichnet,**
daß die Platten (2a) eine Abstützfläche (2c) aufweist, die mit mindestens einem Verankerungsdübel (2b) fest verbunden ist und eine Oberflächengestaltung (2m) aufweist, die durch ein Gitter an Vertiefungen oder Vorsprüngen gekennzeichnet ist.

9. Vorrichtung nach Anspruch 1,
dadurch **gekennzeichnet,**
daß die Platte (2a) vorgesehen ist, um auf der Fläche (2k), die der Abstützfläche (2c) gegenüberliegt, einen Einsatz (3) aufzunehmen, der aus einem Kunststoff- oder Keramikmaterial besteht und eine Gelenkfläche (3a) aufweist.

10. Vorrichtung nach Anspruch 9,
dadurch **gekennzeichnet,**
daß der Einsatz (3) in eine Schale (2p, 2'p) eingerastet ist.

11. Vorrichtung nach Anspruch 10,
dadurch **gekennzeichnet,**
daß die Schale (2p) von einem Rand (2n) umgeben ist, der zwei parallele Abschnitte (2g, 2r) aufweist, die jeweils zum Inneren der Schale (2p) gerichtete Rippen (2s, 2t) aufweisen.

12. Vorrichtung nach Anspruch 10,
dadurch **gekennzeichnet,**
daß der Einsatz (3) einen vorstehenden Teil (3c) und eine nachgiebige Fase (3d) zum Einrasten im Inneren der Schale (2p) der Platte (2a) aufweist.

13. Vorrichtung nach Anspruch 10,
dadurch **gekennzeichnet,**
daß der Einsatz (3) einen vorstehenden Teil (3'c) in einer Form aufweist, die komplementär zu der Schale (2'p) ist, während die Schale (2'p) zum Einrasten des Einsatzes (3) eine nachgiebige Fase (2u) aufweist.
